# EUROPEAN PATENT SPECIFICATION

(11) **EP 1 392 186 B1**
(45) Date of publication and mention of the grant of the patent: **15.09.2010**
(21) Application number: 02739601.9
(22) Date of filing: 31.05.2002
(51) Int. Cl.: A61B 17/34

(54) **BALLOON CANNULA WITH OVER-CENTER CLAMP**
BALLONKÄNULE MIT SELBSTHEMMENDER KLEMMBEFESTIGUNG
CANULE A BALLONNET A CLAMP DECENTRE

(30) Priority: 31.05.2001 US 294966 P
(43) Date of publication of application: 03.03.2004
(73) Proprietor: Tyco Healthcare Group LP, Norwalk, CT 06856 (US)
(72) Inventor: PEARTREE, Kenneth, Los Altos, CA 94024 (US); TANAKA, Shigeru, Half Moon Bay, CA 94019 (US)
(74) Representative: HOFFMANN EITLE
(86) International application number: PCT/US2002/017359
(87) International publication number: WO 2002/096307

(56) References cited:
- EP-A- 0 589 452
- WO-A-00/61016
- WO-A-01/26724
- US-A- 5 364 367
- US-A- 5 716 369

## Description

### BACKGROUND

### 1. Technical Field

The technical field relates to skin seals and anchoring cannulas and, more particularly, to a skin seal with a lock assembly able to secure the skin seal about a shaft of the anchoring cannula.

### 2. Description of Related Art

During laparascopic procedures, cannulas are utilized to provide an access port for surgical instruments and a conduit for introducing insulfation gases into the body cavity. Typically, a sharp trocar is positioned within the cannula and utilized to puncture or pierce the tissue or abdominal wall. Thereafter the trocar is removed, leaving the cannula in place and insufflation gases forced into the body cavity to form an anatomical operating space.

In order to prevent the cannula from migrating in or out through the incision, some cannulas are typically provided with anchoring structure to prevent the cannula from slipping out of the incision. However, unless the anchoring structure is firmly secured against the tissue, leakage of the insufflation gases may occur. Thus, it would be desirable to have an anchoring cannula with a skin seal to secure the cannula to the tissue and prevent leakage of insufflation gases.

WO 01/26924 A discloses a balloon dissection apparatus having a skin seal in which a latch assembly is provided engageable with a locking collar such that movement of the latch assembly from a first position to a second position reduces an inner diameter of the locking collar.

WO 00/61016 A discloses a device having a flexible member extending distally, useful for understanding the background of the present invention. Further, WO 00/61016 A discloses a skin seal for use with a cannula comprising a frame having a locking collar and a flange on a distal end of the locking collar, and a latch assembly engageable with the locking collar such that movement of the latch assembly from a first position to a second position reduces an inner diameter of the locking collar.

### SUMMARY

According to the present invention, there is provided a skin seal for use with a cannula as recited in claim 1 below.

In embodiments of the present invention, the flange is able to secure against tissue and prevent leakage. The over center design typically includes a lever and a cam bar for securing the locking collar about the shaft of the cannula. Preferably, the flexible member is a compressible or deformable pad for sealing against the tissue about an incision. The flexible member may comprise other structures such as for example an elastomeric member of bellows construction or other structure configured to seal an incision through the skin.

In relation to the present invention, and usable in conjunction with embodiments of the same, there is also disclosed an anchoring cannula including a cannula body having an internal seal and a hollow shaft extending distally from the cannula body. An anchoring member is affixed to the end of the shaft and movable between a relaxed position and an expanded state to secure the distal end of the cannula within the body.

An embodiment of the skin seal of the present invention is provided about the shaft of the cannula and is movable along the cannula so as to compress the flexible member against an outer surface of the tissue.

The latch assembly is provided to lock the skin in position on the shaft.

In relation to the present invention, and usable in conjunction with embodiments of the same, there is also disclosed a method of securing a cannula to tissue which includes providing the disclosed cannula and an embodiment of the skin seal of the present invention. The cannula is inserted through an incision in the tissue to position the expandable member on a first side of the tissue and the expandable member thereafter expanded. As noted above, the expandable member is preferably an inflatable balloon. Thereafter, the skin seal is moved distally along the shaft of the cannula until the flexible member, preferably a foam pad, is compressed against an outer surface of the skin about the tissue. Thereafter, the latch assembly can be utilized to lock the skin seal about the cannula shaft.

### BRIEF DESCRIPTION OF THE DRAWINGS

In order to better understand the present invention, and to show how the same may be carried into effect, reference will be made, by way of example only, to the accompanying drawings, in which:
FIG. 1 is a perspective view of an exemplary balloon cannula and an embodiment of an associated skin seal according to the present invention;
FIG. 2 is a side perspective view of the balloon cannula and skin seal;
FIG. 3 is a sectional view, taken along line A-A of FIG. 2, of the skin seal illustrating an over center clamp of the skin seal;
FIG. 4 is a top view of a skin seal with the over center clamp in the open position;
FIG. 5 is a view similar to FIG. 4 with the clamp in the closed position;
FIG. 6 is a cross sectional view, of the balloon cannula and skin seal;
FIG. 7 is a perspective view of the balloon cannula of FIG. 1 in use;
FIG. 8 is a perspective view of an alternate configuration of an over center clamp;
FIG. 9 is a side view, partially shown in section, of alternative of a counter tension pad having a bellows construction;
FIG. 10 is a side view, partially shown in section, of one configuration of a counter tension pad having a semi-rigid construction;
FIG. 11 is a side view, partially show in section, of one configuration of a counter tension pad having an elastomeric bumper construction;
FIG. 12 is a side view, partially shown in section, of one configuration of a counter tension pad having a elastomeric spring construction; and
FIG. 13 is a side view, partially shown in section, of one configuration of a counter tension pad having a joy stick construction.

### DETAILED DESCRIPTION OF PREFERRED EMBODIMENTS

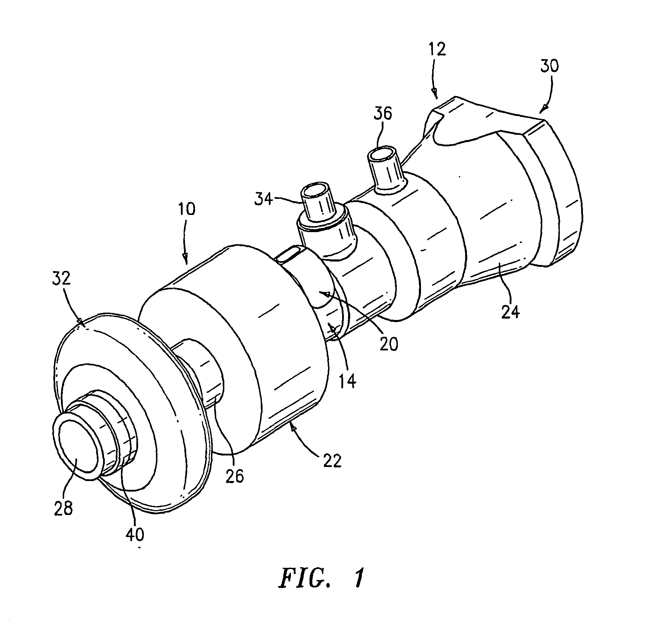

Referring now to FIG. 1, a first embodiment of a skin seal 10 is illustrated for use with an associated balloon cannula 12. Skin seal 10 is provided to seal against the outer surface of an abdominal wall to prevent leakage of insufflation gasses. While skin seal 10 is illustrated being used with balloon cannula 12, it should be understood that various embodiments of the skin seals disclosed herein can be used with other style cannulas to provide a secure seal against the abdominal wall and prevent leakage of insulation gases.

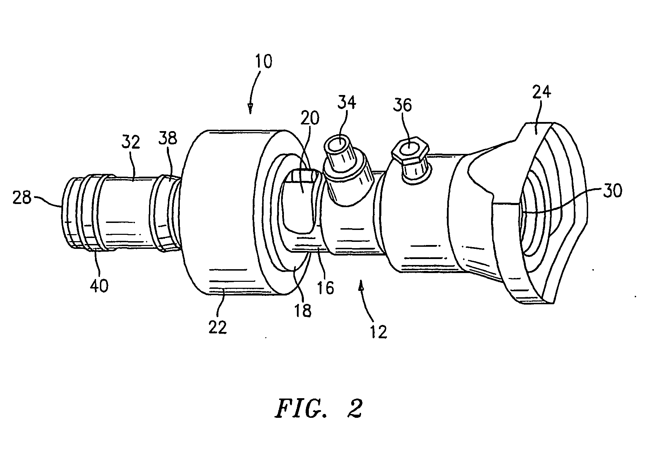

Referring to FIGS. 1 and 2 skin seal 10 generally includes a frame 14 including a locking collar 16 and a mounting flange 18 extending radially outward and distally from locking collar 16. A latch assembly 20 is provided on locking collar 16 to secure skin seal 10 at a location along a shaft of cannula 12 as described herein below. A counter tension pad 22 is affixed to the distal side of flange 18 and is compressible against the abdominal wall to provide a secure seal.

Balloon cannula 12 generally includes a cannula body 24 having an elongated cannula shaft 26 extending distally from body 24. Shaft 26 has a distal opening 28 and body 24 has a proximal opening 30 (FIG. 2) for receipt of operating instruments therethrough. An anchoring balloon 32 is provided on a distal end of shaft 26 and is in fluid communication with an inflation port 34 provided on body 24. Body 24 also includes an insuflation port 36 which is in fluid communication with the interior of shaft 26 so as to provide insuflation fluid into the body. Ports 34 and 36 are configured to receive a syringe containing inflation fluid, such as, for example, saline, so as to inflate anchoring balloon 32 or provide insufflation fluid to the interior of shaft 26. Prefferably, anchoring balloon 32 is secured to shaft 26 by locking rings 38 and 40 located on the proximal and distal sides of anchoring balloon 32, respectively.

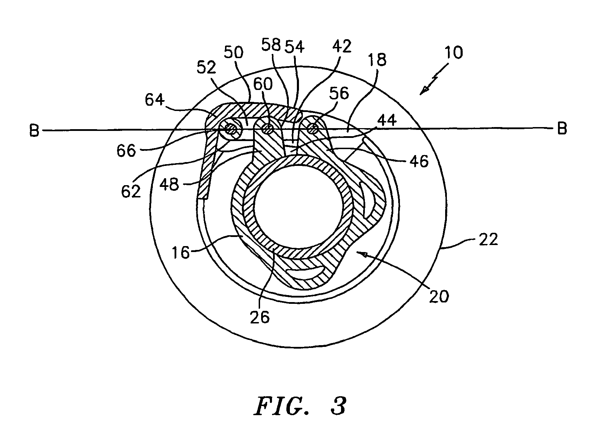

Referring now to FIG. 3, the details of latch assembly 20 to secure locking collar 16, and thus skin seal 10, along shaft 26 will now be described. As shown locking collar 16 is not completely circumferential but defines a split 42 which allows locking collar 16 to be slightly flexible and compressable against shaft 26. Flange 18 defines a similar split 44. Mounting projections 46 and 48 are formed on either side of split 42. Latch assembly 20 is of the "over center clamp" design and generally includes a lever 50 and a cam bar 52. Lever 50 is pivotally connected at a first end 54 to mounting projection 46 by a pin 56 and cam bar 52 is pivotally connected at a first end 58 to mounting projection 48 by a pin 60. A second end 62 of cam bar 52 is pivotally connected to a central portion 64 of lever 50 by a pin 66.

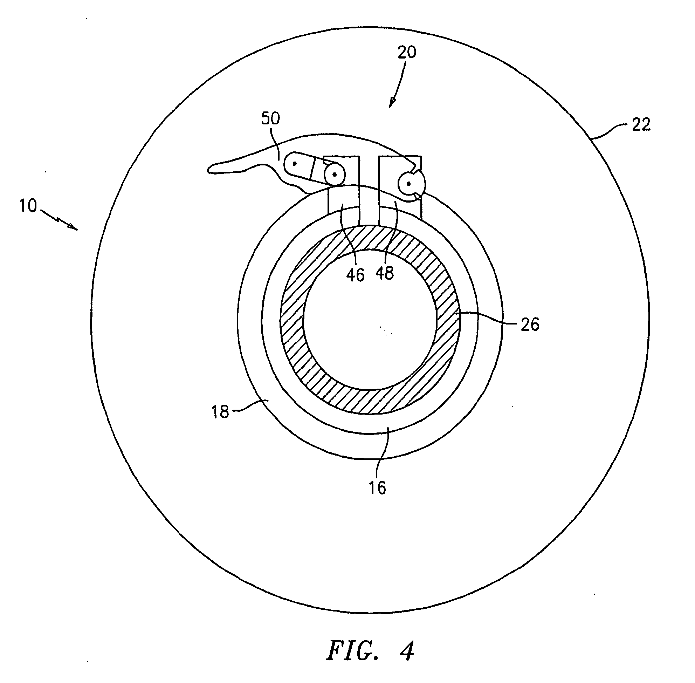

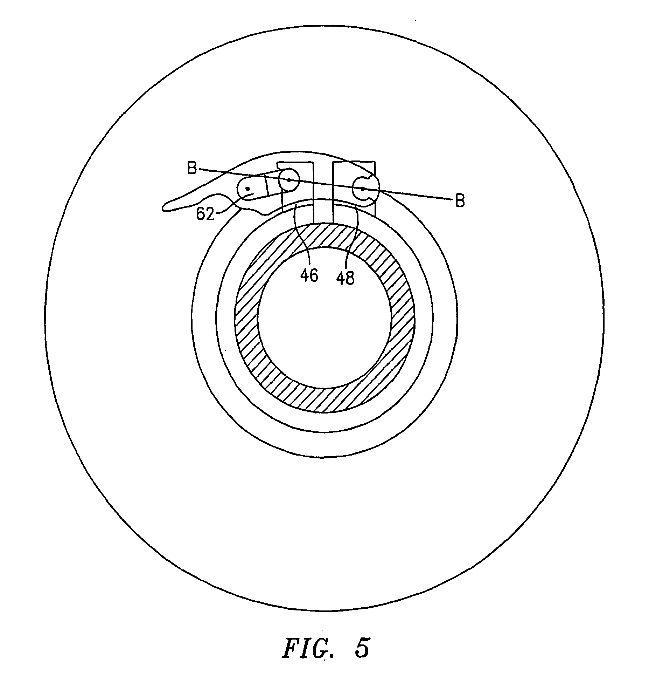

The over center clamping action of latch assembly 20 will now be described with reference to FIGS. 3-5. Referring initially to FIG. 4, when lever 50 is in a generally clockwise most position the distance between mounting projections 46 and 48 are at maximum and locking collar 16 is free to slide along shaft 26. As lever 50 is rotated counterclockwise second end 62 of cam bar 52 moves through an arc and drives mounting projection 48 towards mounting projection 46 to compress against shaft 26. When pin 66 is in level alignment along line B-B with pins 56 and 58 (FIG. 3), projections 46 and 48 are at their closest distance exerting maximum force against shaft 26. As second end 62 moves below line B-B, i.e. over center, and the distance between projections 46 and 48 is slightly increased (FIG. 5). This increase in distance means that slightly increased pressure on lever 50 as it is rotated clockwise is needed to unlock collar 16 from shaft 26 and ensures against inadvertent release of latch assembly 20.

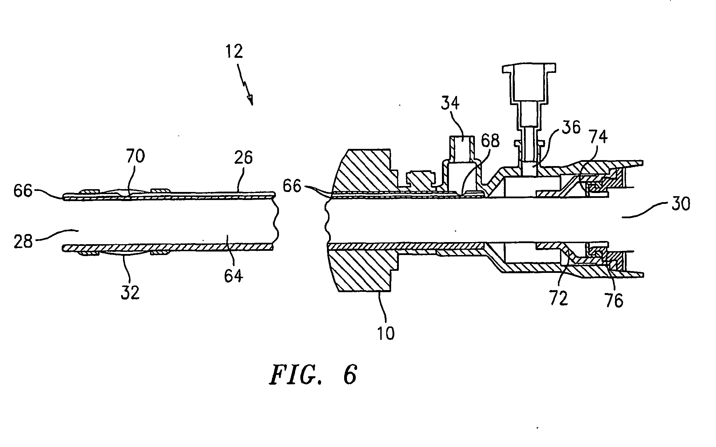

Referring now to FIG. 6, balloon cannula 12 generally defines a through bore 64 extending between distal opening 28 and proximal opening 30. As noted above, insufflation port 36 is in fluid communication with through bore 64 to provide insufflation fluid to a body cavity. Shaft 26 defines an inflation lumen 66 along its length which extends between an arpeture 68 adjacent inflation port 34 and an aperature 70 in fluid communication with an interior of anchoring balloon 32. A duck bill type seal 72 is provided inside cannula body to seal against instruments inserted therethrough and provide a seal against insufflation fluid in the body in the absence of instruments. Preferably, an additional flat apetured seal 74 is provided on a mounting member 76.

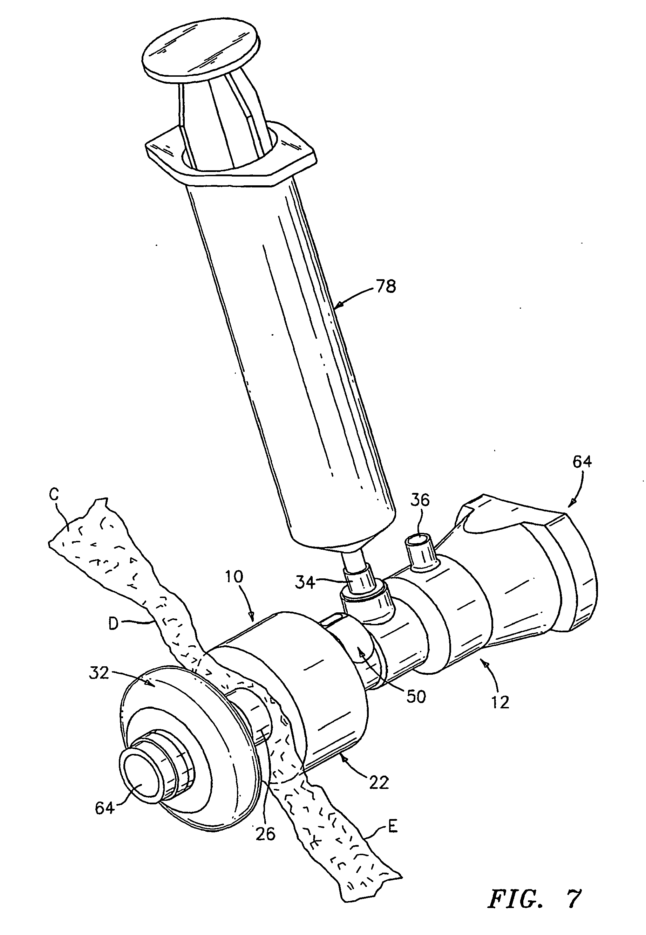

Referring to FIG. 7, the use of skin seal 10 and balloon cannula 12 will now be described. Initially, anchoring balloon 32 is in a deflated condition and skin seal 10 is in a proximal position on shaft 26. A sharp tip trocar (not shown) is inserted through through bore 64 and is used to puncture the abdominal wall such that anchoring balloon is positioned inside the abdominal wall C. The trocar is then removed and a bulb or syringe 78 filled with inflation fluid, such as saline, is inserted in inflation port 34. Fluid is forced through port 34 through aperture 68, through lumen 66 and aperture 70 so as to inflate anchoring balloon 32. Proximal tension is applied to cannula 12 to pull inflated balloon tight against an inner surface D of abdominal wall C and skin seal 10 is moved distally along shaft 26 until counter tension pad 22 is compressed against an outer surface E of abdominal wall C. Then lever 50 is pivoted counter clockwise to the over center position to lock skin seal 10 in place along shaft 26.

Once cannula 12 and skin seal 10 are secured to abdominal wall C, an insulflation fluid or gas may by forced through insufflation port 36 and through through bore 64 and into the body cavity. After an operation has been performed, the insufflation gas is withdrawn through insufflation port 36 and syringe 78 may be used to deflate anchoring balloon 32 allowing cannula 12 removed from abdominal wall C. It may be useful in some instances to release latch assembly 20 prior to deflating anchoring balloon 32.

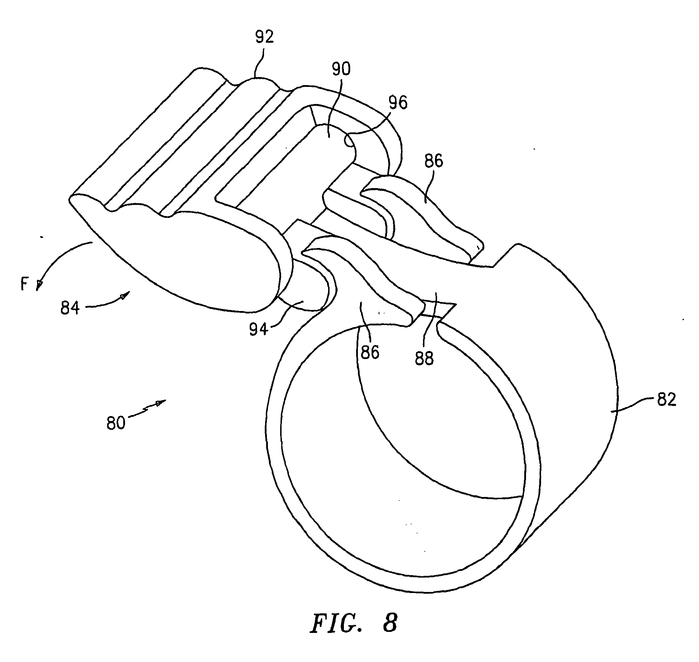

Referring to FIG. 8, there is disclosed a two component over center lock design for use with a skin seal similar to skin seal 10 above. Two part lock 80 includes a clamping band 82 and a latch 84. Band 82 is split similar to collar 16 above and incudes mounting projections 86 at one end of band 82 and a T shaped extension 88 extending from an opposite end of band 82. T shaped extension 88 terminates in a cross-wise enlarged pin 90. Latch 84 generally includes a latch body 92 and integral mounting pin 94 which is engagable with projections 86. As shown, pin 90 is engagable with recesses 96 formed in latch body 92. Pivoting latch body 92 in the direction of arrow F draws T shaped extension 88 and thus the associated end of band 82 closed towards the opposed end of band 82 thereby ensuring a secure seal about an associated cannula shaft.

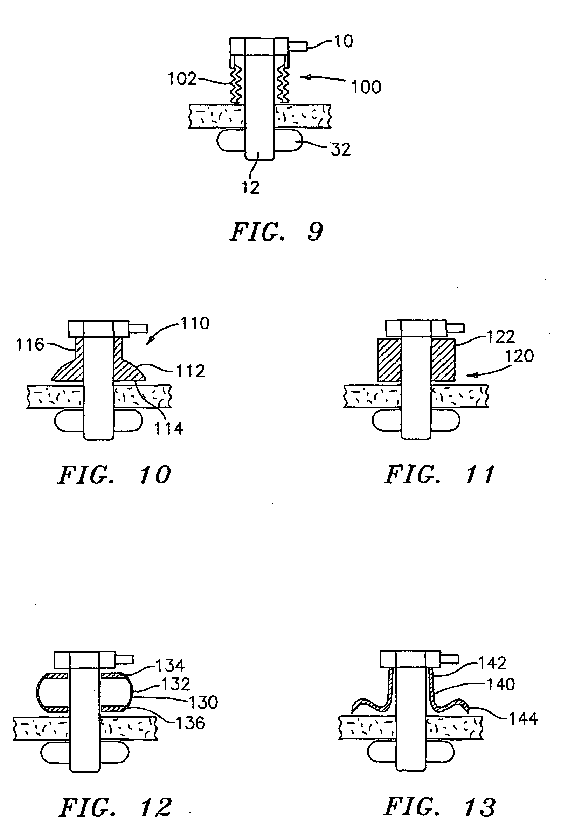

Referring now to FIGS. 9-13, there is illustrated skin seal 10 and balloon cannula 12 as described hereinabove with utilizing the over-center lock design and various materials or forms of construction for the associated counter tension pad. While FIGS. 5-9 disclose the above identified skin seal 10 and balloon cannula 13, the remaining discussion will focus on the alternate counter tension pads. One skilled in the art would know how to affix the various counter tension pads to flange 18 of skin seal 10.

Referring to FIG. 9, there is disclosed a bellows pad 100 which has a flexible bellows type wall 102 formed of a flexible silicone or polyurethane material. Pad 100 can be compressed to maintain compression about the incision and allow side to side flexible movement of a cannula shaft.

Referring now to FIG. 10, there is disclosed a counter tension pad 110 having a semi-rigid pad portion 112 which may be formed of silicone or equivalent. Pad 110 provides a large surface contact area 114 with the skin to prevent migration of pad 110 into the incision. A thin wall 116 may be provided on an upper section of pad 110 to allow for some side to side movement of the cannula shaft.

Referring now to FIG. 11, there is disclosed a further alternative configuration of a counter tension pad 120 utilizing an elastomeric bumper or pad 122. Preferable materials of construction include porous urethane or equivalent for pad 120. Pad 120 provides a soft flexible interface with the skin that maintains compression against the skin for a tight seal and may allow for some side to side manipulation of the cannula shaft.

A further configuration of a counter tension pad 130 is disclosed in FIG. 12 wherein pad 130 may be comprised of an elastomeric spring which includes a thin wall section 132 around its circumference and connected to upper and lower mounting rings 134, 136. Thin wall section 132 acts as a leaf spring under compression to maintain a constant pressure of the pad against the skin.

Referring now to FIG. 13, there is disclosed a further alternative configuration of a counter tension pad 140 which may be formed of silicone or equivalent material. Pad has a "joy stick" construction with a flexible shaft portion 142 and a flexible flange portion 144. Pad 140 utilizes the inherent spring characteristics in the materials to maintain compression against the skin and ensure a wide constant contact area even at extreme manipulation angles of the shaft of the cannula with respect to the skin.

It will be understood that various modifications can be made to the embodiments disclosed herein. For example, other materials of construction for the counter tension pad as well as other types of over center clamp designs may be provided. Therefore, the above description should not be construed as limiting but merely as exemplifications of preferred embodiments. Those skilled in the art will envision other modifications within the scope of the claims appended hereto.

## Claims

1. A skin seal for use with a cannula comprising:
a frame (14) having a locking collar (16) and a flange (18) on a distal end of the locking collar (16);
a latch assembly (20) engageable with the locking collar (16) such that movement of the latch assembly (20) from a first position to a second position reduces an inner diameter of the locking collar (16),
wherein the latch assembly (20) is movable from the first position to a third position such that the inner diameter of the locking collar (16) is slightly increased over that of the locking collar (16) with the latch assembly in the second position, and wherein the latch assembly (20) is an over the center type clamp; and
a flexible member (22) extending from a distal surface of the flange (18).

2. The skin seal as recited in claim 1, wherein the locking collar (16) defines a split such that movement of the latch assembly (20) from the first position to the second position reduces the size of the split.

3. The skin seal as recited in either of claims 1 or 2, wherein the latch assembly (20) includes a lever (50) and a cam bar (52) pivotally mounted to the lever (50).

4. The skin seal as recited in claim 3, wherein a first end (54) of the lever (50) is movably mounted to a first projection (46) on the locking collar (16).

5. The skin seal as recited in either of claims 3 or 4, wherein a first end (58) of the cam bar (52) is movably mounted to a second projection (48) on the locking collar (16).

6. The skin seal as recited in any preceding claim, wherein the flexible member (22) is a deformable pad.

7. The skin seal as recited in any preceding claim, wherein the flexible member (22) is an elastomeric member.

8. The skin seal as recited in any preceding claim, wherein the flexible member (22) has a bellows construction.

9. The flexible member as recited in any preceding claim, wherein the flexible member (22) allows for off axis movement of a cannula (12) inserted therethrough.

## Patentansprüche

1. Hautabdichtung zur Verwendung mit einer Kanüle, umfassend:
einen Rahmen (14) mit einem Feststellkragen (16) und einem Flansch (18) an einem distalen Ende des Feststellkragens (16);
eine Riegelanordnung (20), die mit dem Feststellkragen (16) derart in Eingriff bringbar ist, dass eine Bewegung der Riegelanordnung (20) aus einer ersten Position in eine zweite Position einen inneren Durchmesser des Feststellkragens (16) reduziert,
wobei die Riegelanordnung (20) aus der ersten Position in eine dritte Position derart bewegbar ist, dass der innere Durchmesser des Feststellkragens (16) etwas über den des Feststellkragens (16) mit der Riegelanordnung in der zweiten Position vergrößert wird, und wobei die Riegelanordnung (20) eine Klemme vom Über-die-Mitte-Typ ist; und
ein flexibles Element (22), das sich von einer distalen Oberfläche des Flansches (18) erstreckt.

2. Hautabdichtung nach Anspruch 1, wobei der Feststellkragen (16) einen Spalt derart definiert, dass eine Bewegung der Riegelanordnung (20) aus der ersten Position in die zweite Position die Größe des Spalts reduziert.

3. Hautabdichtung nach einem der Ansprüche 1 oder 2, wobei die Riegelanordnung (20) einen Hebel (50) und eine Nockenstange (52), die schwenkbar an dem Hebel (50) montiert ist, enthält.

4. Hautabdichtung nach Anspruch 3, wobei ein erstes Ende (54) des Hebels (50) beweglich an einem ersten Vorsprung (46) an dem Feststellkragen (16) montiert ist.

5. Hautabdichtung nach einem der Ansprüche 3 oder 4, wobei ein erstes Ende (58) der Nockenstange (52) beweglich an einem zweiten Vorsprung (48) an dem Feststellkragen (16) montiert ist.

6. Hautabdichtung nach einem der vorhergehenden Ansprüche, wobei das flexible Element (22) ein deformierbares Polster ist.

7. Hautabdichtung nach einem der vorhergehenden Ansprüche, wobei das flexible Element (22) ein Elastomerelement ist.

8. Hautabdichtung nach einem der vorhergehenden Ansprüche, wobei das flexible Element (22) eine Faltenbalgkonstruktion aufweist.

9. Flexibles Element nach einem der vorhergehenden Ansprüche, wobei das flexible Element (22) eine Bewegung einer Kanüle (12), die **dadurch** eingesetzt ist, abseits der Achse ermöglicht.

## Revendications

1. Joint pour la peau pour utilisation avec une canule comprenant:
un châssis (14) comportant un collier de verrouillage (16) et une bride (18) sur une extrémité distale du collier de verrouillage (16);
un ensemble de verrouillage (20) pouvant être mis en prise avec le collier de verrouillage (16) de sorte que le mouvement de l'ensemble de verrouillage (20) d'une première position à une seconde position réduit un diamètre interne du collier de verrouillage (16),
où l'ensemble de verrouillage (20) est déplaçable de la première position à une troisième position de façon que le diamètre interne du collier de verrouillage (16) soit légèrement augmenté par rapport à celui du collier de verrouillage (16) avec l'ensemble de verrouillage dans la seconde position, et où l'ensemble de verrouillage (20) est un clamp du type décentré; et
un élément flexible (22) s'étendant d'une surface distale de la bride (18).

2. Joint pour la peau selon la revendication 1, dans lequel le collier de verrouillage (16) définit une fente de telle sorte que le mouvement de l'ensemble de verrouillage (20) de la première position à la seconde position réduit la taille de la fente.

3. Joint pour la peau selon l'une quelconque des revendications 1 ou 2, dans lequel l'ensemble de verrouillage (20) comporte un levier (50) et une barre à came (52) montée d'une manière pivotante sur le levier (50).

4. Joint pour la peau selon la revendication 3, dans lequel une première extrémité (54) du levier (50) est montée d'une manière mobile sur une première saillie (46) sur le collier de verrouillage (16).

5. Joint pour la peau selon l'une quelconque des revendications 3 ou 4, dans lequel une première extrémité (58) de la barre à came (52) est montée d'une manière mobile sur une deuxième saillie (48) sur le collier de verrouillage (16).

6. Joint pour la peau selon l'une quelconque des revendications précédentes, dans lequel l'élément flexible (22) est un coussinet déformable.

7. Joint pour la peau selon l'une quelconque des revendications précédentes, dans lequel l'élément flexible (22) est un élément élastomère.

8. Joint pour la peau selon l'une quelconque des revendications précédentes, dans lequel l'élément flexible (22) a une construction en soufflet.

9. Elément flexible selon l'une quelconque des revendications précédentes, dans lequel l'élément flexible (22) permet un mouvement désaxé d'une canule (12) insérée à travers celui-ci.
